# EUROPEAN PATENT SPECIFICATION

(11) **EP 1 099 453 B1**
(45) Date of publication and mention of the grant of the patent: **01.06.2005**
(21) Application number: 00310045.0
(22) Date of filing: 10.11.2000
(51) Int. Cl.: A61M 25/00

(54) **Injection catheter**
Injektionskatheter
Cathéter à injection

(30) Priority: 12.11.1999 US 165354; 01.05.2000 US 562611; 01.05.2000 US 562612; 01.05.2000 US 563769
(43) Date of publication of application: 16.05.2001
(73) Proprietor: Biosense Webster, Inc., Diamond Bar, California 91765 (US)
(72) Inventor: Ponzi, Dean, Glendora, California 91741 (US)
(74) Representative: Mercer, Christopher Paul

(56) References cited:
- EP-A- 0 900 549
- EP-A- 0 908 194
- EP-A- 0 962 191
- WO-A-99/22655
- DE-A- 19 633 406

## Description

### FIELD OF THE INVENTION

This invention relates to a catheter with an injection needle for infusing therapeutic or diagnostic agents into the tissue of organs.

### BACKGROUND OF THE INVENTION

Targeted delivery of therapeutic or diagnostic agents, such as occurs in gene therapy, is very desirable but often presents a difficult challenge. A potential benefit of targeted delivery is that there is an increased efficiency obtained by the precise placement of the therapeutic agent. There are several problems to his procedure which must be overcome in order to obtain satisfactory results from such therapy, such as the problems of obtaining access to the delivery site, transporting the therapeutic agent to the desired site, injecting the therapeutic agent at the proper depth within the organ tissue, steering the distal end of the catheter to a desired location within the organ prior to infusing the agent, and positioning the distal tip of the catheter at precisely the same location where prior measurements have indicated that the drug should be infused. It is also important to for a physician to be able to monitor the position of the infusion needle with respect to the wall of the organ. In the case of an organ, such as the heart, in which the walls are in constant motion, the activity of positioning and monitoring the position of the distal tip of the catheter, or infusion needle, becomes especially difficult.

U.S. Patent No. 3,598,119 discloses a medical device for injecting drugs in which the injection needle is guided through an inner lumen of a catheter for insertion of the needle under skin tissue. A bladder at the distal end of the catheter may be inflated through another lumen for holding the point of the needle point in a fixed position beneath the skin.

U.S. Patent No. 4,578,061 discloses a catheter for injecting a liquid into a vein, or artery, through an injection needle which is longitudinally movable beyond the distal end of the catheter. A dual chamber system is utilized within the catheter tip to provide for movement of a plunger to extend the injection needle and also to allow for a plunger to be used to apply a predetermined dose of medication through the injection needle.

U.S. Patent No. 4,578,061 discloses an injection catheter having a longitudinal movable needle which may be moved through a lumen in order to extend out of the side wall of the catheter for injecting a liquid into a blood vessel. The needle is normally retracted into the device so that the needle will not penetrate tissue as the device is moved through a body duct. Thereafter, the needle is moved out of the side of the catheter into a vessel wall in order to infuse a liquid into the wall of a vessel.

U.S. Patent No. 5,244,460 is directed toward a method for improving blood flow to the heart. More particularly this patent is directed toward a medical procedure for improving the growth of cardiac blood vessels by inserting a catheter into a coronary artery and injecting into the heart a blood vessel growth promoting peptide through an injection port of the catheter.

U.S. Patent No. 5,419,777 is directed toward a catheter for injection of a fluid into body cavities such as coronary vessels and arteries. This patent, as is the case with the '061 patent, illustrates the use of an injection needle which protrudes laterally through the side walls of the distal tip of the catheter. In the case of drug injections to be made into coronary vessels and arteries, it is very desirable to have the needles extend out of the side walls of the catheter and at an acute angle to the walls of the vessel in order to penetrate the walls of the vessel for injection of the agent.

U.S. Patent 5,431,168, assigned to the same assignee as the present patent application, is directed toward a steerable catheter which includes a puller wire for controlling the distal end of the catheter from a control handle which is mounted on the proximal end of the catheter.

EP-A-0 980 226 discloses an injection catheter system for infusing a diagnostic or therapeutic agent into the wall of an organ which includes an electromagnetic sensor disposed within the distal tip of the catheter for providing very precise location information for the distal tip of the catheter.

U.S. Patent Application Serial No. 09/280,202, now published as US 6, 165, 164, discloses an injection catheter where extension of the injection needle is determined by the amount of movement that is allowed in the injection handle. The injection handle is adjustable, permitting the physician to adjust the needle extension to the desired length. Although this design has several advantages, it can cause the needle to buckle inside the catheter due to frictional force between the needle and the housing in which the needle is mounted, which can result in unpredictable needle extension.

EP-A-0962191, which comprises the state of the art under Article 54(3) EPC, discloses an injection catheter comprising a catheter body, a tip section, a needle control handle and an injection needle extending through the tip section, catheter body and needle control handle.

### SUMMARY OF THE INVENTION

This present invention is directed to a catheter for infusing therapeutic or diagnostic agents into the tissue of organs.

In one embodiment the catheter comprises a catheter body comprising a flexible tubing having proximal and distal ends and at least one lumen therethrough. A tip section comprising a flexible tubing having proximal and distal ends is mounted at its proximal end to the distal end of the catheter body. It is understood that the tip section and catheter body can be two separate components fixedly attached together or a single integral tubing wherein the tip section is the distal end of the tubing on which is mounted, for example, the tip electrode and ring electrodes.

A needle control handle is provided at the proximal end of the catheter body, and may or may not be directly mounted to the catheter body. The needle control handle comprises an outer body and a piston. The outer body has a piston chamber therein. One end of the piston is slidably mounted within the piston chamber. A compression spring is provided in the piston chamber between the piston and the outer body. The compression spring can be aligned such that one end of the compression spring is in contact with the outer body, while the other end is in contact with the piston. Alternatively, one end of the compression spring can be fixed relative to the outer body, while the other end of the compression spring is fixed relative to the piston.

An injection needle extends through the tip section, catheter body, and needle control handle. The proximal end of the injection needle is attached to one of the outer body and the piston, and the distal end of the injection needle is contained within the distal end of the tip section when in a retracted position, e.g. when the catheter is being introduced into the patient.

Application of a longitudinal force to one of the outer body and the piston causes longitudinal movement of the other of the outer body and the piston, thereby compressing the compression spring. This results in distal movement of the injection needle relative to the catheter body so that the distal end of the injection needle extends outside the distal end of the tip section.

Further aspects of the invention are set out in the accompanying dependent claims 2 to 29.

### DESCRIPTION OF THE DRAWINGS

These and other features and advantages ofthe present invention will be better understood by reference to the following detailed description when considered in conjunction with the accompanying drawings wherein:
Figure 1 is a side plan view of one embodiment of the catheter of the present invention.
Figure 2a is a side cross-sectional view of the needle control handle where the needle is in a retracted position.
Figure 2b is a side cross-sectional view of the needle control handle where the needle is in an extended position.
Figure 3 is a side cross-sectional view of the catheter section showing an embodiment having three lumens and showing the position of the electromagnetic mapping sensor and the injection needle.
Figure 4 is a side cross-sectional view of the catheter tip section showing an embodiment having three lumens and showing the position of the electromagnetic mapping sensor and the puller wire.
Figure 5 is a side cross-sectional view of the catheter body, including the junction between the catheter body and the catheter tip section.
Figure 6 is a transverse cross-sectional view of the catheter tip section along line 6-6 showing an embodiment having three lumens.
Figure 7 is a transverse cross-sectional view of the catheter body along line 7-7.
Figure 8 is a side cross-sectional view of the catheter handle;
Figure 9 is a side cross-sectional view of a tip section according to the invention having three lumens, showing the injection needle and a needle stop.
Figure 9A is an enlarged view of the needle stop of Figure 9
Figure 10 is a schematic partial side cross-sectional view of a tip section showing an alternative needle stop design in accordance with the invention.
Figure 11 is a schematic partial side cross-sectional view of a tip section showing another alternative needle stop design in accordance with the invention.
Figure 12 is a side cross-sectional view of a tip section according to the invention having three lumens, showing the position of the electromagnetic mapping sensor and the injection needle.
Figures 12A, 12B, and 12C are end cross-sectional views of alternative embodiments of injection needles in accordance with the invention.

### DETAILED DESCRIPTION

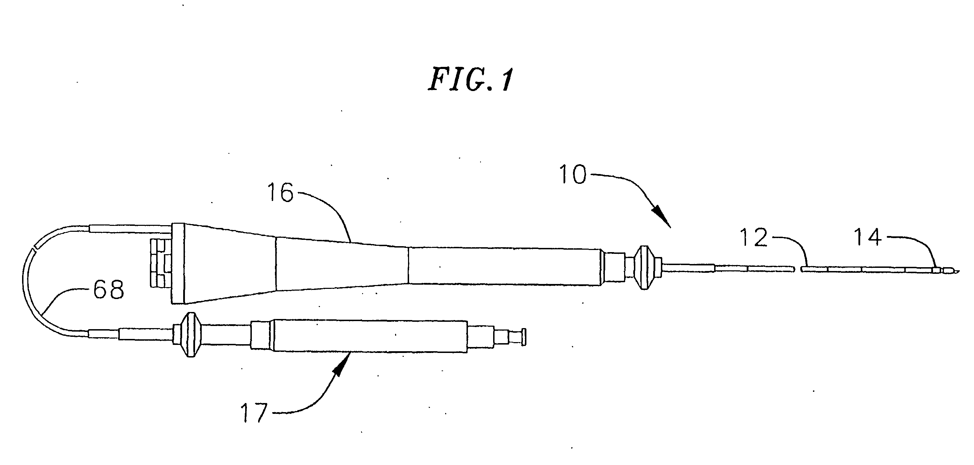

In a preferred embodiment of the invention, there is provided a catheter for use for injection of a therapeutic or diagnostic agent into the heart. As shown in Figure 1, catheter 10 comprises an elongated catheter body 12 having proximal and distal ends, a tip section 14 at the distal end of the catheter body 12, a deflection control handle 16 at the proximal end of the catheter body 12, and a needle control handle 17 attached indirectly to the catheter body proximal the deflection control handle.

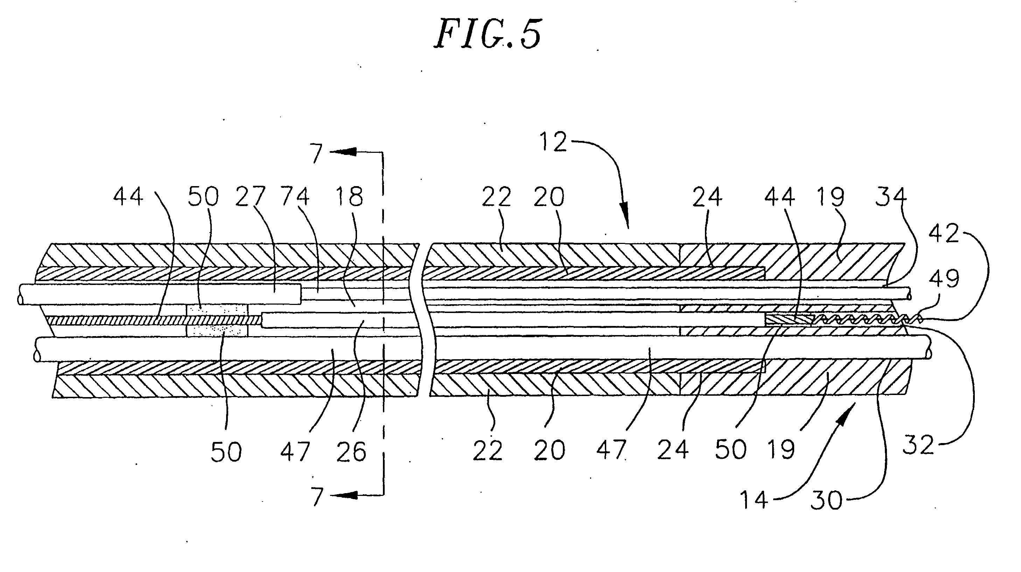

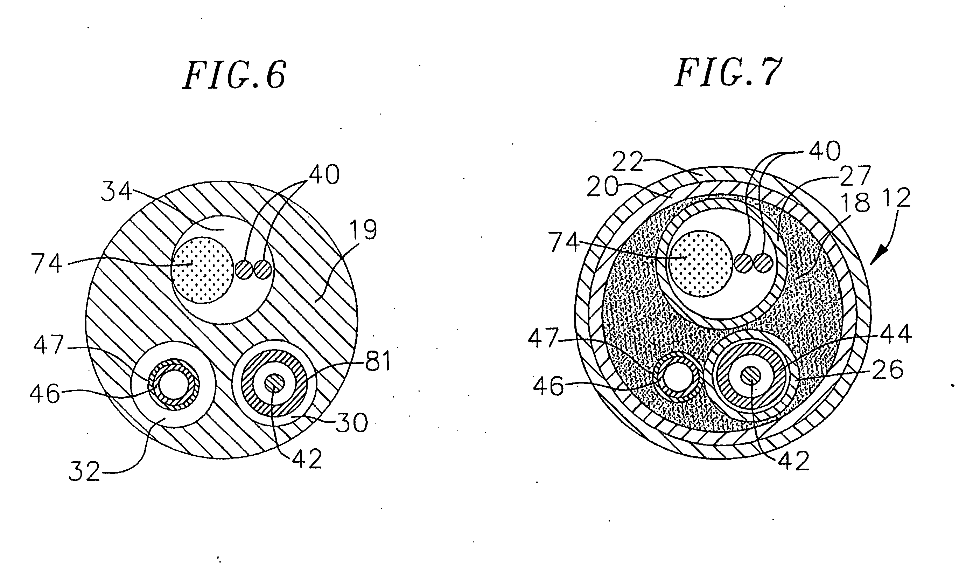

With reference to Figures 5 and 7, the catheter body 12 comprises a single, central or axial lumen 18. The catheter body 12 is flexible, i.e., bendable, but substantially non-compressible along its length. The catheter body 12 may be of any suitable construction and made of any suitable material. A presently preferred construction comprises an outer wall 22 made of a polyurethane or nylon. The outer wall 22 comprises an imbedded braided mesh of stainless steel or the like to increase torsional stiffness of the catheter body 12 so that, when the control handle 16 is rotated, the tip section of the catheter 10 will rotate in a corresponding manner.

The outer diameter of the catheter body 12 is not critical, but is preferably no more than about 2.67 mm (8 French). Likewise the thickness of the outer wall 22 is not critical. The inner surface of the outer wall 22 is lined with a stiffening tube 20, which can be made of any suitable material, preferably polyimide. The stiffening tube, along with the braided outer wall 22, provides improved torsional stability while at the same time minimizing the wall thickness of the catheter, thus maximizing the diameter of the single lumen. The outer diameter of the stiffening tube 20 is about the same as or slightly smaller than the inner diameter of the outer wall 22. Polyimide tubing is presently preferred because it may be very thin walled while still providing very good stiffness. This maximizes the diameter of the central lumen 18 without sacrificing strength and stiffness. Polyimide material is typically not used for stiffening tubes because of its tendency to kink when bent. However, it has been found that, in combination with an outer wall 22 of polyurethane, nylon or other similar material, particularly having a stainless steel braided mesh, the tendency for the polyimide stiffening tube 20 to kink when bent is essentially eliminated with respect to the applications for which the catheter is used.

A particularly preferred catheter has an outer wall 22 with an outer diameter of about 2.34 mm (0.092 inch) and an inner diameter of about 1.60 mm (0.063 inch) and a polyimide stiffening tube having an outer diameter of about 1.56 mm (0.0615 inch) and an inner diameter of about 1.32 mm (0.052 inch).

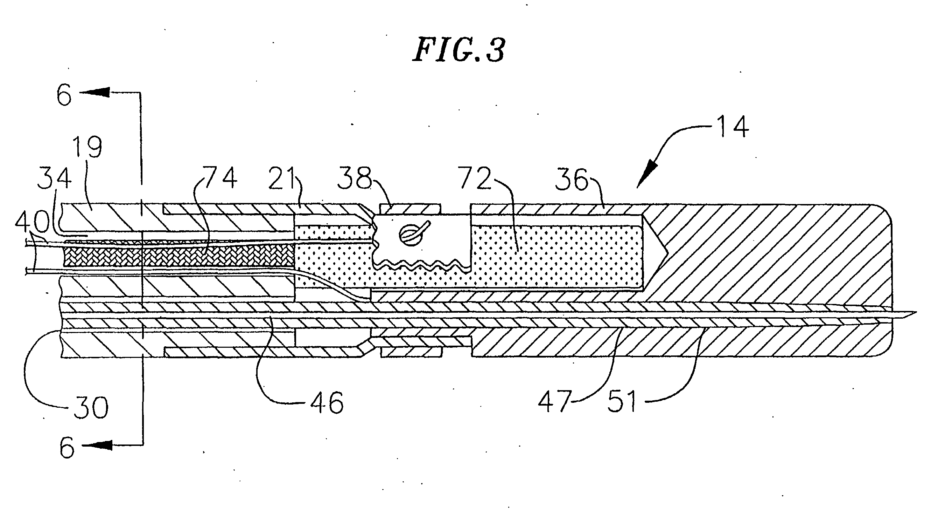

As shown in Figures 3, 4 and 6, the tip section 14 comprises a short section of tubing 19 having three lumens 30,32 and 34. The tubing 19 is made of a suitable non-toxic material which is preferably more flexible than the catheter body 12. A presently preferred material for the tubing 19 is braided polyurethane, i.e., polyurethane with an embedded mesh of braided stainless steel or the like. The outer diameter of the tip section 14, like that of the catheter body 12is preferably no greater than about 2.67 mm (8 French). The size of the lumens is not critical. In a particularly preferred embodiment, the tip section has an outer diameter of about 2.33 mm (7 French (.092 inch)) and the first lumen 30 and second lumen 32 are generally about the same size, having a diameter of about 0.56 mm (0.022 inch), with the third lumen 34 having a slightly larger diameter of about 0.91 mm (0.036 inch).

A preferred means for attaching the catheter body 12 to the tip section 14 is illustrated in Figure 5. The proximal end of the tip section 14 comprises an inner counter bore 24 that receives the outer surface of the polyimide stiffening tube 20. The tip section 14 and catheter body 12 are attached by glue or the like.

The stiffening tube 20 is held in place relative to the outer wall 22 at the proximal end of the catheter body 12. In preferred construction of the catheter body 12, a force is applied to the proximal end of the stiffening tube 20 which causes the distal end of the stiffening tube 20 to firmly push against the counter bore 24. While under compression, a first glue joint is made between the stiffening tube 20 and the outer wall 22 by a fast drying glue, e.g. Super Glue®. Thereafter a second glue joint is formed between the proximal ends of the stiffening tube 20 and outer wall 22 using a slower drying but stronger glue, e.g., polyurethane.

As shown in Figure 7, extending through the central lumen 18 of the catheter body 12 are lead wires 40, an injection needle 46, a sensor cable 74, and a compression coil 44 through which a puller wire 42 extends. A central lumen 18 catheter body is preferred over a multi-lumen body because it has been found that the central lumen 18 body permits better tip control when rotating the catheter 10. The central lumen 18 permits the lead wires 40, the injection needle 46, the sensor cable 74, and the puller wire 42 surrounded by the compression coil 44 to float freely within the catheter body. If such wires and cables were restricted within multiple lumens, they tend to build up energy when the control handle 16 is rotated, resulting in the catheter body 12 having a tendency to rotate back if, for example, the handle is released, or if bent around a curve, to flip over, either for which are undesirable performance characteristics.

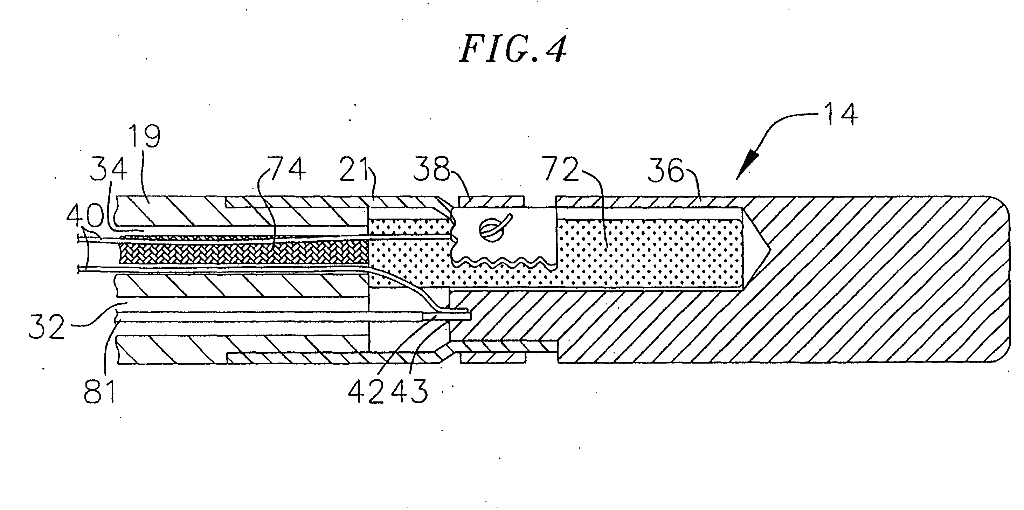

With reference to Figures 3 and 4, mounted at the distal end of the tip section 14 is a tip electrode 36. Preferably the tip electrode 36 has a diameter about the same as the outer diameter of the tubing 19. The tip electrode 36 is connected to the tubing 19 by means of a plastic housing 21, preferably made of polyetheretherketone (PEEK). The proximal end of the tip electrode 36 is notched circumferentially and fits inside the distal end of the plastic housing 21 and is bonded to the housing 21 by polyurethane glue or the like. The proximal end of the plastic housing 21 is bonded with polyurethane glue or the like to the distal end of the tubing 19 of the tip section 14. Alternatively, the tip electrode 36 can be mounted directly to the distal end of the flexible tubing 19 of the tip section 14.

Mounted on the distal end of the plastic housing 21 is a ring electrode 38. The ring electrode 38 is slid over the plastic housing 21 and fixed in place by glue or the like. If desired, additional ring electrodes may be used and can be positioned over the plastic housing 21 or over the flexible tubing 19 of the tip section 14.

The tip electrode 36 and ring electrode 38 are each connected to separate lead wires 40. The lead wires 40 extend through the third lumen 34 of tip section 14, the catheter body 12, and the control handle 16, and terminate at their proximal end in an input act (not shown) that may be plugged into an appropriate monitor (not shown). If desired, the portion of the lead wires 40 extending through the catheter body 12, control handle 16 and proximal end of the tip section 14 may be enclosed or bundled within a protective tube or sheath.

The lead wires 40 are attached to the tip electrode 36 and ring electrode 38 by any conventional technique. Connection of lead wire 40 to the tip electrode 36 is preferably accomplished by weld 43, as shown in Figure 4.

A puller wire 42 is provided for deflection of the tip section 14. The puller wire 42 is anchored at its proximal end to the control handle 16 and anchored at its distal end to the tip section 14. The puller wire 42 is made of any suitable metal, such as stainless steel or Nitinol, and is preferably coated with Teflon@ or the like. The coating imparts lubricity to the puller wire 42. The puller wire 42 preferably has a diameter ranging from about 0.15 mm (0.006 inches) to about 0.25 mm (0.010 inches).

The compression coil 44 extends from the proximal end of the catheter body 12 to the proximal end of the tip section 14. The compression coil 44 is made of any suitable metal, preferably stainless steel. The compression coil 44 is tightly wound on itself to provide flexibility, i.e., bending, but to resist compression. The inner diameter of the compression coil 44 is preferably slightly larger than the diameter of the puller wire 42. For example, when the puller wire 42 has a diameter of about 0.18 mm (0.007 inches), the compression coil 44 preferably has an inner diameter of about 0.20 mm (0.008 inches). The Teflon® coating on the puller wire 42 allows it to slide freely within the compression coil 44. Along its length, the outer surface ofthe compression coil 44 is covered by a flexible, non-conductive sheath 26 to prevent contact between the compression coil 44 and any of the lead wires 40, injection needle 46 or sensor cable 74. A non-conductive sheath 26 made of polyimide tubing is presently preferred.

As shown in Figures 4 and 5, the compression coil 44 is anchored at its proximal end to the proximal end of the stiffening tube 20 in the catheter body 12 by glue to form a glue joint 50 and at its distal end to the tip section 14 in the second lumen 32. The glue may be applied by means of a syringe or the like through a hole made between the outer surface of the catheter body 12 and the single lumen.

The puller wire 42 extends into the second lumen 32 of the tip section 14. The distal end of the puller wire 42 is anchored to the tip electrode 36 or to the side of the catheter tip section 14. With reference to Figure 4 and 5, within the tip section 14, and distal to the glue joint 50, the turns of the compression coil are expanded longitudinally. Such expanded turns 49 are both bendable and compressible and preferably extend for a length of about 12.7 mm (0.5 inch). The puller wire 42 extends through the expanded turns 49 then into a plastic, preferably Teflon®, sheath 81, which prevents the puller wire 42 from cutting into the wall of the tip section 14 when the tip section 14 is deflected.

An injection needle 46 is provided, which extends from the needle control handle through the catheter body 12, through the first lumen 30 of the tip section 14 and through a passage 51 in the tip electrode 36. In one embodiment, the injection needle 46 is formed of Nitinol, and, as illustrated in Figure 3, is preferably formed with a beveled edge at the distal tip of the needle. Alternatively, the injection needle 46 is formed of plastic, or a portion of the needle is formed of plastic and another portion of the needle is formed of metal. The needle 46 is coaxially mounted within a protective tube 47, preferably made of polyimide, which serves to prevent the needle from buckling and also serves to electrically insulate the needle from the distal electrode 36. The protective tube 47 additionally serves to provide a fluid-tight seal surrounding the injection needle 46. Figure 3 depicts the injection needle 46 extending beyond the distal end of the tip electrode 36, as it would be positioned in order to infuse diagnostic or therapeutic fluid into the human heart. The distal end of the injection needle 46 is withdrawn into the tip electrode 36 during the period of time that the catheter is inserted through the vasculature of the body and also during the period of time in which the catheter is removed from the body to avoid injury. Alternatively, the tip section 14 can be provided without a tip electrode 36, in which case the distal end of the injection needle 46 could be retracted into the first lumen 30 of the tip section 14.

The injection needle 46 is made from one or more straight pieces of small diameter tubing having an outer diameter that allows the tubing to fit within the catheter. Preferably the injection needle 46 has an inner diameter ranging from about 0.18 mm (0.007 inch) to about 0.28 mm (0.011 inch) and an outer diameter ranging from about 0.30 mm (0.012 inch) to about 0.41 mm (0.016 inch). Preferably the injection needle 46 has a total length ranging from about 1.65 m (65 inches) to about 2.16 m (85 inches), more preferably about 1.91 m (75 inches).

In one embodiment, at least one of the pieces of tubing is plastic tubing. For example, the entire injection needle 46 can consist of a single piece of plastic tubing. In another embodiment, the injection needle 46 comprises two or more discrete pieces of plastic tubing bonded together, end to end, by glue or the like. In another embodiment, the injection needle comprises a piece of plastic tubing bonded or otherwise attached at one of its ends to the end of a piece of metal tubing.

The tubing, whether plastic or metal, preferably has straight position memory so that when it is bent to a small radius, its natural tendency is to spring back to the straight position. This property is particularly important for the tubing that forms the distal region of the injection needle (i.e., the portion of the needle within the tip section 14), because when the tip section is deflected, the needle will deflect with the tip. When the tip section is then straightened from its deflected position, the memory of the tubing forming the distal region of the needle pushes the tip section back towards the straight position to the same axis as the catheter body.

Additionally, the tubing, particularly the tubing used for the distal region of the injection needle, is preferably made of a biocompatiable material that is capable of being beveled. The material for the tubing preferably also has a low coefficient of friction, a good surface finish for slidability within the catheter, and the ability to be cleaned and sterilized. The good surface finish also reduces coagulate build-up on the needle. The tubing, particularly the tubing used at the proximal end of the infusion needle, preferably also has the ability to bond or fuse to an adapter for infusion.

A particularly preferred plastic for use in the present invention is PEEK (polyetheretherketone), although other suitable plastics such as polycarbonate, polyimide, fiberglass, and composites thereof could also be used. Suitable metals for use in connection with the present invention include Nitinol and stainless steel, although Nitinol is preferred for use at the distal region of the needle due to its shape-memory properties. If desired, at least a portion of the tubing, such as the portion that forms the distal region of the needle, is provided with a lubricious coating, such as Teflon® or silicone, preferably having a thickness ranging from about 7.62 µm (0.0003 inch) to about 50.8 µm (0.002 inch). In another alternative embodiment, a biocompatible lubricant, such as mineral oil is injected around the needle once assembled.

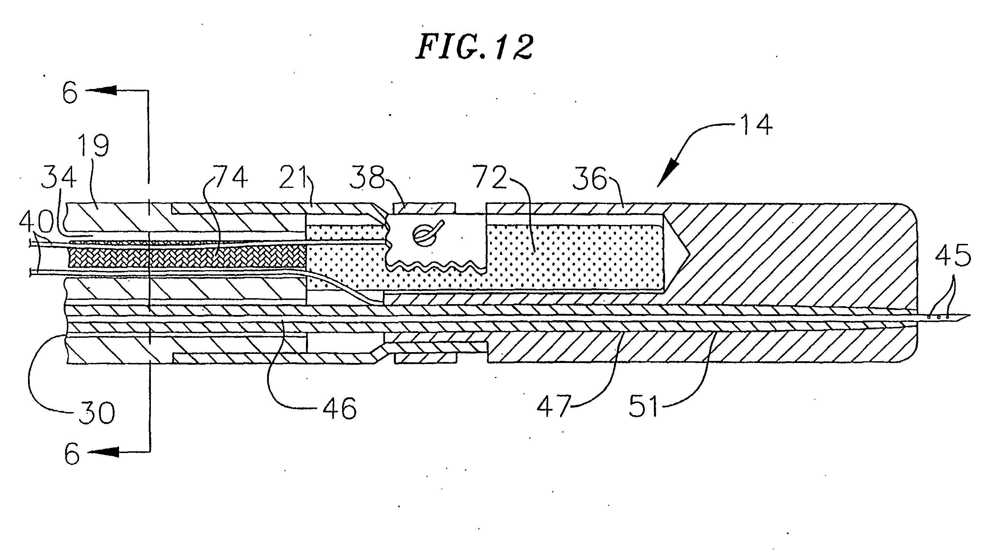

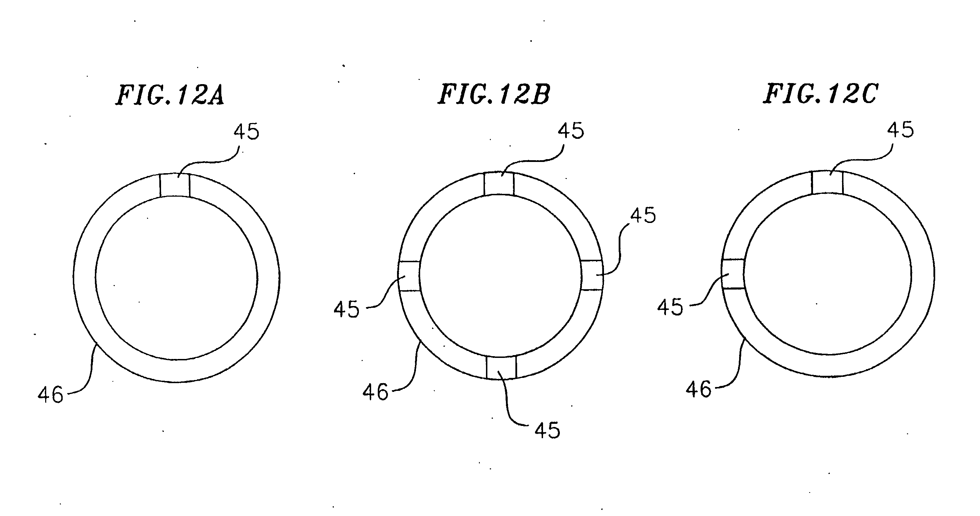

In another embodiment, as depicted in Figure 12, the distal region of the injection needle 46, i.e., that portion of the needle that is extendable beyond the distal end of the tip section 14, is provided with one or more fluid ports 45 along its length, through which fluid can also pass. The fluid ports 45 can be of any suitable shape, such as round, oval, or rectangular (e.g., vertical or horizontal slots), and can be formed by any suitable method, such as by laser drilling. The fluid ports 45 can be provided on only one side of the needle 46, as shown in Figure 12A, about the circumference of the needle, as shown in Figure 12B, or only on selected sides of the needle, as shown in Figure 12C. Preferably at least 3 fluid ports are provided, more preferably at least 5 fluid ports are provided. Preferably each fluid port 45 has a length ranging from about 0.13 mm (0.005 inch) to about 1.02 mm (0.04 inch), more preferably about 0.51 mm (0.02 inch). In a particularly preferred embodiment, four fluid ports are provided, with the most distal fluid port positioned a distance of about 2 inches from the distal end of the needle, and with a distance of about 0.51 mm (0.02 inch) provided between the fluid ports. In this preferred embodiment, two fluid ports are provided on one side of the needle, with the other two fluid ports on the opposite side of the needle. The fluid ports 45 enhance the ability of the drug or other agent passing through the needle to weep into the injection side and be more evenly distributed, allowing for better absorption of the agent by the heart tissue.

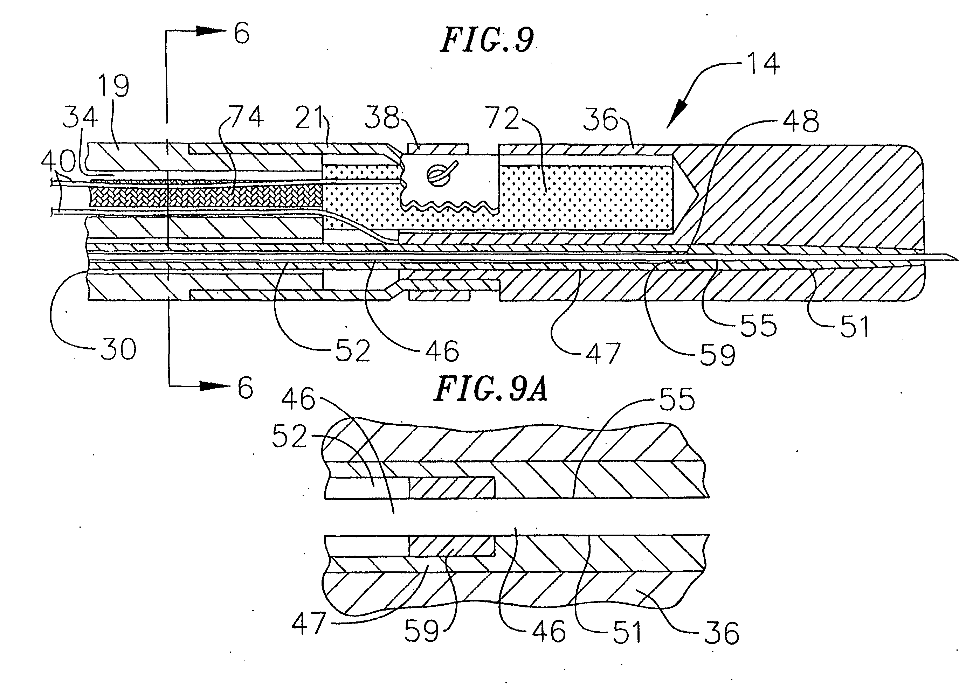

In one embodiment, as depicted in Figures 9 and 9A, the inner region of the protective tube 47 serves as a needle passage. The needle passage has a proximal region 52 with a proximal diameter and a distal region 55 with a distal diameter that is less than the proximal diameter. The distal diameter is approximately equal to the outer diameter of the injection needle 46 to provide a fluid-tight seal, as described above. This design creates a step 48 at the distal end of the proximal region 52. A needle stop 59 is mounted on a portion of the injection needle 46 that is positioned within the proximal region 52 of the needle passage. The needle stop 59 is sized to prevent passage of the portion of the injection needle on which the needle stop is mounted from passing into the distal region 55 of the needle passage. In the embodiment depicted in Figures 9 and 9A, the needle stop 59 is in the form of a collar that is mounted in surrounding relation to the injection needle 46. The collar extends only a short length along the needle, e.g., a distance ranging from about 0.25 mm (0.01 inch) to about 19.1 mm (0.75 inch).

This design limits the distance that the injection needle 46 can extend beyond the distal end of the tip section 14. As described in more detail below, needle extension and retraction is accomplished with the needle control handle. When the injection needle 46 is moved distally using the needle control handle, it pushes the needle stop 59 distally into the step 48 at the distal end of the proximal region 52. The interaction between the needle stop 59 and the step 48 prevents further distal movement of the injection needle 46, thereby limiting the extent to which the needle can extend beyond the distal end of the tip section 14. This design ensures that the needle always extends out of the tip section 14 the same distance, regardless of whether the tip section is straight or deflected.

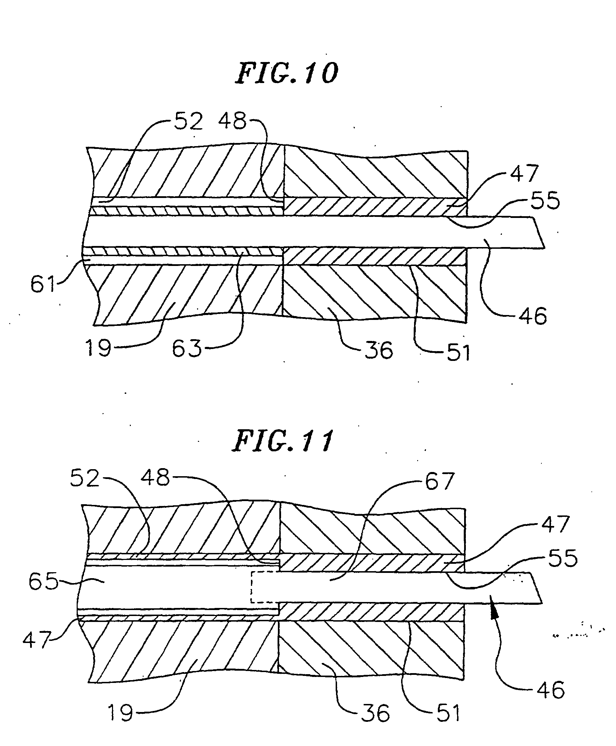

Figure 10 depicts an alternative design for the needle passage and needle stop. In this embodiment, a lumen 61 in the tubing 19 of the tip section 14 serves as the proximal region 52 of the needle passage. As in the embodiment of Figures 9 and 9A, a passage 51 is provided in the tip electrode 36. Within the tip electrode 36, the needle 46 is coaxially mounted within a protective tube 47, although the protective tube does not extend into the lumen 61. The inner region of the protective tube 47 serves as the distal region 55 of the needle passage. The inner diameter of the lumen 61 is thus larger than the inner diameter of the protective tube 47, thus creating a step 48 at the distal end of the lumen. An elongated tube 63, preferably having a length of at least about 50.8 mm (2 inches), is coaxially mounted around a portion of the injection needle 46 that is in the proximal region 52. The elongated tube 63 can be made of any suitable material, such as plastic or Nitinol. The elongated tube 63 has an diameter greater than the inner diameter of the protective tube 47, and thus serves as a needle stop in a manner similar to the collar described above.

The needle stop 59 need not be coaxially mounted on the injection needle 46. For example, the needle stop could be in the form of a rectangular tab that is mounted on only a portion of the circumference of the injection needle 46. Any other suitable design can be provided for the needle stop 59, so long as the needle stop is of a size and shape so that it cannot pass into the distal region 55 of the needle passage.

In another alternative design, as shown in Figure 11, the needle stop 59 is integral with the injection needle 46. In this embodiment, the injection needle 46 is formed of two pieces of tubing, a proximal tubing 65 and a distal tubing 67, each of which can be made of any suitable material as described above, such as metal or plastic. The proximal end of the distal tubing 67 is bonded or otherwise attached to the distal end of the proximal tubing 65. Preferably the proximal end of the distal tubing 67 is inserted into the distal end of the proximal tubing 65 and attached by polyurethane glue or the like. The proximal tubing 65 has an outer diameter greater than the outer diameter of the distal tubing 67 and greater than the inner diameter of the distal region 55 of the needle passage. The proximal tubing thus acts as the needle stop 59.

The needle passage can be formed by any suitable combination of elements in the tip section so long as it has a proximal region 52 with a proximal diameter and a distal region 55 with a distal diameter that is less than the proximal diameter. Thus, the needle passage can be formed from a tubing that is separate from the tip section 14, as shown, for example, in Figures 11 and 10, discussed above. Alternatively, the needle passage can be formed directly in the tip section 14 and tip electrode 36, or directly in the tip section only if no tip electrode is provided. In another alternative, a combination of these approaches can be provided, for example, as shown in Figure 10 discussed above.

Additionally, an electromagnetic sensor 72, as depicted in Figure 3, is contained within the distal end of the tip section 14. The electromagnetic sensor 72 is connected to an electromagnetic sensor cable 74, which extends through the third lumen 34 of the tip section 14 through the catheter body 12 into the control handle 16. The electromagnetic sensor cable 74 comprises multiple wires encased within a plastic sheath. In the control handle 16, the wires of the sensor-cable 74 are connected to a circuit board 64. The circuit board 64 amplifies the signal received from the electromagnetic sensor and transmits it to a computer in a form understandable by the computer. Also, because the catheter is designed for single use only, the circuit board contains an EPROM chip which shuts down the circuit board after the catheter has been used. This prevents the catheter, or at least the electromagnetic sensor, from being used twice. A suitable electromagnetic sensor is described, for example, in U.S. Patent No. 4,391,199. A preferred electromagnetic mapping sensor 72 is manufactured by Biosense Ltd. Israel and marketed under the trade designation NOGA. To use the electromagnetic sensor 72, the patient is placed in a magnetic field generated, for example, by situating under the patient a pad containing coils for generating a magnetic field. A reference electromagnetic sensor is fixed relative to the patient, e.g., taped to the patient's back, and the injection catheter containing a second electromagnetic sensor is advanced into the patient's heart. Each sensor comprises three small coils which in the magnetic field generate weak electrical signals indicative of their position in the magnetic field. Signals generated by both the fixed reference sensor and the second sensor in the heart are amplified and transmitted to a computer which analyzes the signals and then displays the signals on a monitor. By this method, the precise location of the sensor in the catheter relative to the reference sensor can be ascertained and visually displayed. The sensor can also detect displacement of the catheter that is caused by contraction of the heart muscle.

Using this technology, the physician can visually map a heart chamber. This mapping is done by advancing the catheter tip into a heart chamber until contact is made with the heart wall. This position is recorded and saved. The catheter tip is then moved to another position in contact with the heart wall and again the position is recorded and saved.

The electromagnetic mapping sensor 72 can be used alone or more preferably in combination with the tip electrode 36 and ring electrode 38, By combining the electromagnetic sensor 72 and electrodes 36 and 38, a physician can simultaneously map the contours or shape of the heart chamber, the electrical activity of the heart, and the extent of displacement of the catheter and hence identify the presence and location of the ischemic tissue. Specifically, the electromagnetic mapping sensor 72 is used to monitor the precise location of the tip electrode in the heart and the extent of catheter displacement. The tip electrode 36 and ring electrode 38 are used to monitor the strength of the electrical signals at that location. Healthy heart tissue is identified by strong electrical signals in combination with strong displacement. Dead or diseased heart tissue is identified by weak electrical signals in combination with dysfunctional displacement, i.e., displacement in a direction opposite that of healthy tissue. Ischemic, or hibernating or stunned, heart tissue is identified by strong electrical signals in combination with impaired displacement. Hence, the combination of the electromagnetic mapping sensor 72 and tip and ring electrodes 36 and 38 is used as a diagnostic catheter to determine whether and where to infuse a drug into the wall of the heart. Once the presence and location of ischemic tissue has been identified, the tip section 14 of the catheter can be deflected so that the injection needle 46 is generally normal, i.e., at a right angle, to the ischemic tissue, and the injection needle may then be extended out of the distal end of the tip electrode 36 and into the wall of the heart.

It is understood that, while it is preferred to include both electrophysiology electrodes and an electromagnetic sensor in the catheter tip, it is not necessary to include both. For example, an injection catheter having an electromagnetic sensor but no electrophysiology electrodes may be used in combination with a separate mapping catheter system. A preferred mapping system includes a catheter comprising multiple electrodes and an electromagnetic sensor, such as the NOGA-STAR catheter marketed by Biosense Webster, Inc., and means for monitoring and displaying the signals received from the electrodes and electromagnetic sensor, such as the Biosense-NOGA system, also marketed by Biosense Webster, Inc.

The electrode lead wires 40 and electromagnetic sensor cable 74 must be allowed some longitudinal movement within the catheter body so that they do not break when the tip section 14 is deflected. As shown in Figure 5, to provide for such lengthwise movement, there is provided a tunnel through the glue joint 50, which fixes the proximal end of the compression coil 44 inside the catheter body 12. The tunnel is formed by a transfer tube 27, preferably made of a short segment of polyimide tubing. The transfer tube is approximately 60 mm long and has an outer diameter of about 0.53 mm (.021 inch) and an inner diameter of about 0.48 mm (.019 inch).

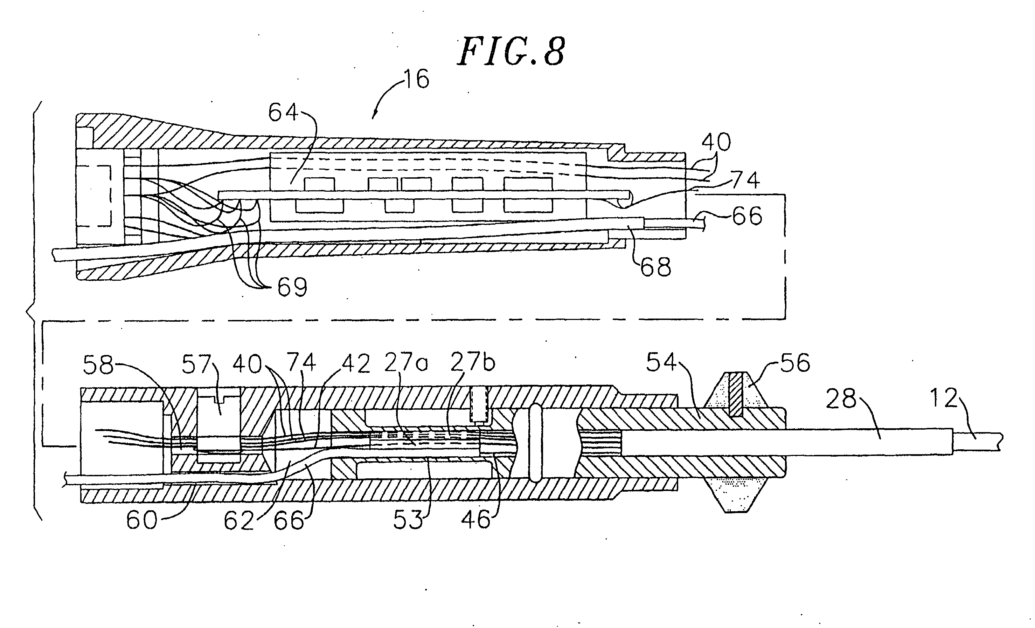

Longitudinal movement of the puller wire 42 relative to the catheter body 12, which results in deflection of the tip section 14, is accomplished by suitable manipulation of the control handle 16. As shown in Figure 8, the distal end of the control handle 16 comprises a piston 54 with a thumb control 56 for manipulating the puller wire 42. The proximal end of the catheter body 12 is connected to the piston 54 by means of a shrink sleeve 28.

The injection needle 46 within the protective tube 47, the puller wire 42, the lead wires 40 and the electromagnetic sensor cable 74 extend through the piston 54. The puller wire 42 is anchored to an anchor pin 57 located proximal to the piston 54. The lead wires 40 and electromagnetic sensor cable 74 extend through a first tunnel 58, located near the side of the control handle 16. The electromagnetic sensor cable 74 connects to the circuit board 64 in the proximal end of the control handle. Wires 69 connect the circuit board 64 to a computer and imaging monitor (not shown).

The injection needle 46 and protective tube 47 extend through a guide tube 66, preferably made of polyurethane, and are afforded longitudinal movement therein. The guide tube 66 is anchored to the piston 54, preferably by glue at glue joint 53. This design allows the needle 46 and protective tube 47 longitudinal movement within the control handle 16 so that the needle 46 does not break when the piston 54 is adjusted to manipulate the puller wire 42. Within the piston 54, the puller wire 42 is situated within a transfer tube 27a, and the electromagnetic sensor cable 74 and lead wires 40 are situated within another transfer tube 27b to allow longitudinal movement of the wires and cable near the glue joint 53.

The injection needle 46, protective tube 47 and guide tube 66 extend through a second tunnel 60 situated near the side of the control handle 16 opposite the anchor pin 57. To avoid undesirable bending of the injection needle 46, a space 62 is provided between the proximal end of the piston 54 and the distal end of the second tunnel 60. Preferably the space 62 has a length of at least 12.7 mm (0.50 inch) and more preferably about from about 15.2 mm (0.60 inch) to about 22.9 mm (0.90 inch).

In the proximal end of the control handle 16, the injection needle 46, protective tube 47 and polyurethane guide tube 66 extend through a second larger plastic guide tube 68, preferably made of Teflon®, which affords the guide tube 66, injection needle 46, and protective tube 47 longitudinal slidable movement. The second guide tube 68 is anchored to the inside of the control handle 16 by glue or the like and extends proximally beyond the control handle 16. The second guide tube 68 protects the injection needle 46 both from contact with the circuit board 64 and from any sharp bends as the guide tube 66, needle 46, and protective tube 47 emerge from the control handle 16.

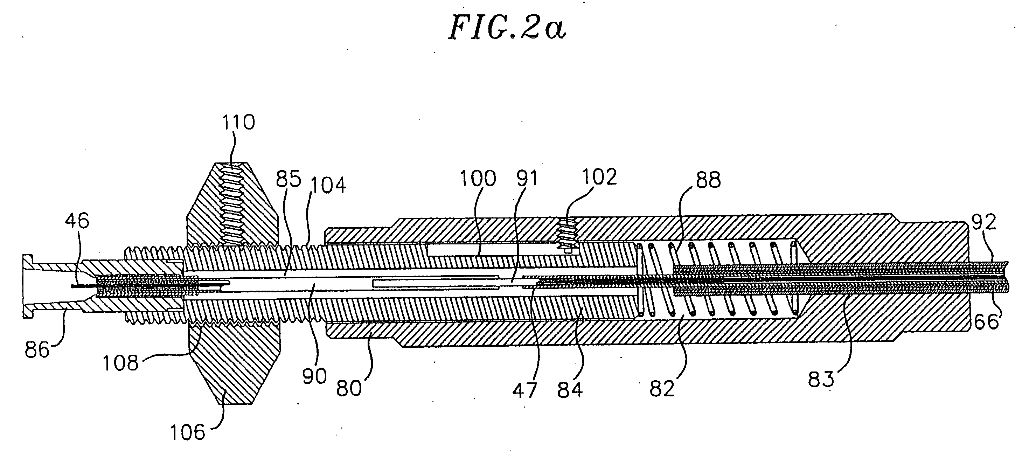

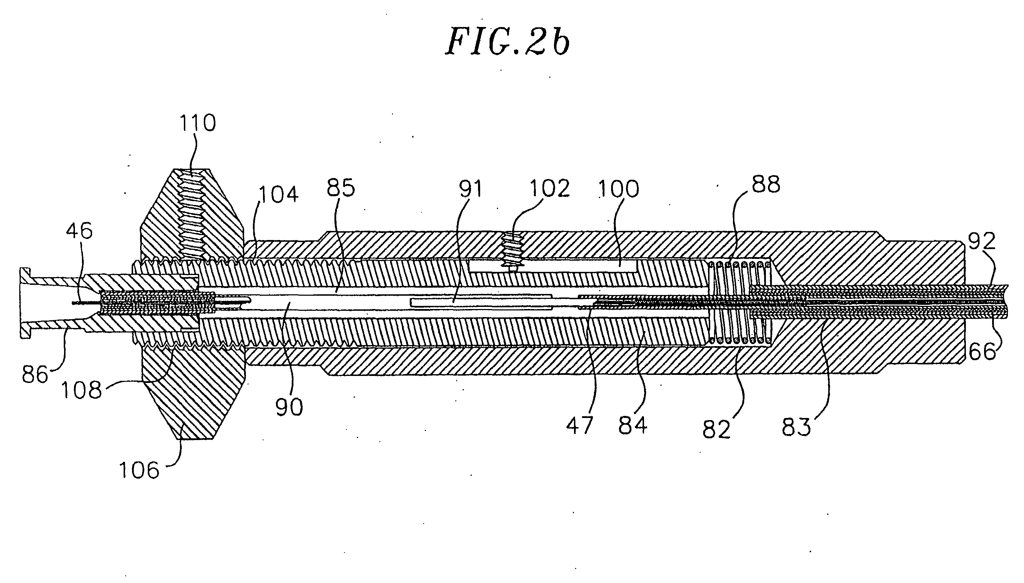

Extension and retraction of the injection needle 46 out the distal end of the tip electrode 36 is accomplished by the needle control handle 17. As illustrated in Figures 2a and 2b, in one embodiment, the needle control handle 17 comprises a generally cylindrical outer body 80 having proximal and distal ends, a piston chamber 82 extending a part of the way therethrough, and a needle passage 83 extending a part of the way therethrough. The piston chamber 82 extends from the proximal end of the handle part way into the body 80, but does not extend out the distal end of the body. The needle passage 83, which has a diameter less than that of the piston chamber 82, extends from the proximal end of the piston chamber to the proximal end of the outer body 80.

A piston 84, having proximal and distal ends, is slidably mounted within the piston chamber 82. A Luer connector 86 is mounted in the distal end of the outer body. The piston 84 has an axial passage 85 through which the injection needle 46 extends, as described in more detail below. A compression spring 88 is mounted within the piston chamber 82 between the distal end of the piston 84 and the outer body 80. The compression spring 88 can either be arranged between the piston 84 and outer body 80, or can have one end in contact with or fixed to the piston 84, while the other end is in contact with or fixed to the outer body 80.

The proximal end of the injection needle 46 is mounted either directly or indirectly to the Luer connector 86 by means of a first rigid tube 90, preferably made of stainless steel, which has a proximal end fitted into the Luer connector. This arrangement fixedly attaches the injection needle 46 to the piston 84 so that it moves longitudinally with the piston. The first rigid tube 90 is also fixedly attached either directly or indirectly to the piston 84 and moves longitudinally with the piston. In the embodiment illustrated in Figures 2a and 2b, the first rigid tube 90 is attached to the proximal end of the piston 84, alternatively the first rigid tube 90 can be attached anywhere along the length of the piston 84. The injection needle 46 and first rigid tube 90 extend through the axial passage 85 of the piston 84. Within the axial passage 85, a second rigid tube 91, preferably made of stainless steel, has a proximal end mounted coaxially within or surrounding the distal end of the first rigid tube 90. The proximal end of the second rigid tube 91 is mounted within the protective tube 47, which has its proximal end inside the axial passage 85, and the distal end of the second rigid tube is attached, directly or indirectly, to the outer body 80. The guide tube 66, through which the protective tube 47 and injection needle 46 extend, as discussed above, is fixedly attached to the outer body 80 by means of a shrink sleeve 92, as is generally known in the art.

In use, force is applied to the piston 84 to cause distal movement of the piston relative to the outer body 80, which compresses the compression spring 88. This movement causes the injection needle 46 to correspondingly move distally relative to the outer body, guide tube 66, protective tube 47 and catheter body 12, so that the distal end of the injection needle extends outside the distal end of the tip electrode 36. When the force is removed from the piston, the compression spring 88 pushes the piston 84 proximally to its original position, thus causing the distal end of the injection needle 46 to retract back into the tip electrode 36. Upon distal movement of the piston 84, the first rigid tube 90 moves distally over the second rigid tube 91 to prevent the injection needle 46 from buckling within the axial passage 85.

The piston 84 further comprises a longitudinal slot 100 extending along a portion of its outer edge. A securing means, such as a set screw, pin, or other locking mechanism 102 extends through the outer body 80 and into the longitudinal slot 100. This design limits the distance that the piston can be slid proximally out of the piston chamber 82. When the distal end of the injection needle 46 is in the retracted position, preferably the securing means 102 is at or near the distal end of the longitudinal slot 100.

The proximal end of the piston 84 has a threaded outer surface 104. A circular thumb control 106 is rotatably mounted on the proximal end of the piston. The thumb control 106 has a threaded inner surface 108 that interacts with the threaded outer surface 104 of the piston. The thumb control 106 acts as a stop, limiting the distance that the piston 84 can be pushed into the piston chamber 82, and thus the distance that the injection needle 46 can be extended out the distal end of the catheter. The threaded surfaces of the thumb control 106 and piston 84 allow the thumb control to be moved closer or farther from the proximal end of the outer body 80 so that the extension distance of the injection needle can be controlled by the physician. A securing means, such as a tension screw 110 is provided in the thumb control 106 to control the tension between the thumb control and piston 84. As would be recognized by one skilled in the art, the thumb control 106 can be replaced by any other mechanism that can act as a stop for limiting the distance that the piston 84 extends into the piston chamber 82, and it is not necessary, although it is preferred, that the stop be adjustable relative to the piston.

In another preferred embodiment constructed in accordance with the present invention, two or more puller wires (not shown) are provided to enhance the ability to manipulate the tip section. In such an embodiment, a second puller wire and a surrounding second compression coil extend through the catheter body and into separate off-axis lumens in the tip section. The lumens of the tip section receiving the puller wires may be in adjacent quadrants. The first puller wire is preferably anchored proximal to the anchor location of the second puller wire. The second puller wire may be anchored to the tip electrode or may be anchored to the wall of the tip section adjacent the distal end of tip section.

The distance between the distal end of the compression coils and the anchor sites of each puller wire in the tip section determines the curvature of the tip section 14 in the direction of the puller wires. For example, an arrangement wherein the two puller wires are anchored at different distances from the distal ends of the compression coils allows a long reach curve in a first plane and a short reach curve in a plane 90° from the first, i.e., a first curve in one plane generally along the axis of the tip section before it is deflected and a second curve distal to the first curve in a plane transverse, and preferably normal to the first plane. The high torque characteristic of the catheter tip section 14 reduces the tendency for the deflection in one direction to deform the deflection in the other direction. Suitable deflection control handles for use with such a catheter are described in EP-A-0 904 796, EP-A-0 985 423, EP-A-0 982 047 and U.S. Patent Application entitled "Single Gear Drive Bidirectional Control Handle for Steerable Catheter" to Tim Bumbalough, et al., filed April 10, 2000, now published as US 6, 468, 260.

As an alternative to the above described embodiment, the puller wires (not shown) may extend into diametrically opposed off-axis lumens in the tip section. In such an embodiment, each of the puller wires may be anchored at the same location along the length of the tip section, in which case the curvatures of the tip section in opposing directions are the same and the tip section can be made to deflect in either direction without rotation of the catheter body.

The preceding description has been presented with reference to presently preferred embodiments of the invention. Workers skilled in the art and technology to which this invention pertains will appreciate that alterations and changes in the described structure may be practiced without meaningful departing from the scope of this invention as defined by the accompanying claims.

Accordingly, the foregoing description should not be read as pertaining only to the precise structures described and illustrated in the accompanying drawings, but rather should be read consistent with and as support to the following claims which are to have their fullest and fair scope.

## Claims

1. An injection catheter (10) comprising:
a catheter body (12) comprising a flexible tubing having proximal and distal ends and at least one lumen (18) therethrough;
a tip section (14) comprising a flexible tubing having proximal and distal ends, wherein the proximal end of the tip section is mounted on the distal end of the catheter body;
a needle control handle (17) at the proximal end of the catheter body (12), the needle control handle comprising:
an outer body (80) having a piston chamber (82) therein;
a piston (84) having two ends, wherein one end of the piston is slidably mounted within the piston chamber (82); and
a compression spring (88) in the piston chamber between the outer body (80) and the piston (84);
an injection needle (46) extending through the tip section (14), catheter body (12), and needle control handle (17) and having a proximal end attached to one of the outer body (80) and the piston (84) and a distal end within the distal end of the tip section (14);
whereby longitudinal force applied to one of the outer body (80) and the piston (84) causes longitudinal movement of the other of the outer body (80) and the piston (84), thereby compressing the compression spring (88), resulting in distal movement of the injection needle (46) relative to the catheter body (12) so that the distal end of the inj ection needle extends outside the distal end of the tip section (14).

2. An injection catheter as claimed in claim 1, wherein the piston (84) further comprises:
a slot (100) extending longitudinally along a portion of its length; and
a securing means (102) extending through at least a portion of the outer body (80) and into the slot (100);
whereby, during operation, the securing means (102) limits longitudinal movement of the piston (84) relative to the outer body (80).

3. An injection catheter as claimed in claim 1, wherein the needle control handle (17) further comprises an axial passage (83, 85) extending along the length of the needle control handle through which the injection needle (46) extends.

4. An injection catheter as claimed in claim 3, wherein the needle control handle (17) further comprises:
a first rigid tube (90) fixedly attached to the piston (84) and extending through the axial passage (83, 85);
a second rigid tube (91) having first and second ends extending through the axial passage (83, 85), wherein the first end of the second rigid tube is further slidably mounted coaxially in or around at least a part of the first rigid tube (90), and the second end of the second rigid tube (91) is fixedly mounted to the outer body (80); and
wherein the injection needle (46) extends through the first rigid tube (90) and second rigid tube (91).

5. An injection catheter as claimed in claim 1, wherein at least a portion of the piston (84) has a threaded outer surface (104), and wherein the needle control handle (17) further comprises a thumb control (106) rotatably mounted on the piston (84) and having a threaded inner surface (108) that interacts with the threaded outer surface (104) of the piston;
whereby rotation of the thumb control (106) about the piston (84) moves the position of the thumb control relative to the piston, and further whereby the position of the thumb control (106) limits the longitudinal movement of the piston (84) relative to the outer body (80).

6. An injection catheter as claimed in claim 5, further comprising a securing means (110) extending radially through the thumb control (106), whereby the securing means (110) can be moved against the threaded outer surface (104) of the piston (84) to secure the position of the thumb control (106) relative to the piston (84).

7. An injection catheter according to any preceding claim, wherein the piston chamber (82) has proximal and distal ends, the distal end of the piston (84) being slidably mounted within the piston chamber (82); wherein the proximal end of the injection needle (46) is attached to the piston, and further wherein the longitudinal force comprises distal force applied to the piston, the distal force causing distal movement of the piston (84) relative to the outer body (80), compressing the compression spring (88), resulting in distal movement of the injection needle (46) relative to the outer body (80), catheter body (12) and tip section (14) so that the distal end of the injection needle (46) extends outside the distal end of the tip section (14), and further whereby, upon removal ofthe distal force, the compression spring (88) expands to exert a force on the piston (84), causing proximal movement of the piston (84) relative to the outer body (80), thereby retracting the distal end of the injection needle (46) into the distal end of the tip section (14).

8. An injection catheter as claimed in claim 7, wherein the compression spring (88) has two ends, wherein one end of the compression spring is in contact with the outer body (80) and the other end of the compression spring is in contact with the piston (84).

9. An injection catheter as claimed in claim 7, wherein the proximal end of the piston (84) extends out of the proximal end of the outer body (80) and comprises a thumbrest (106) to facilitate manual movement of the piston (84) relative to the outer body (80).

10. An injection catheter according to claim 1, wherein the at least one lumen (18) extending through the catheter body (12) comprises
at least one needle passage (47), the needle passage (47) having a proximal region (52) having a proximal diameter and a distal region (55) having a distal diameter less than the proximal diameter, wherein the
injection needle (46) extends through the catheter body (12) and has a distal end within the needle passage (47), the injection needle (46) being longitudinally slidable within the catheter body (12) so that its distal end can extend beyond the distal end of the catheter body (12), the injection catheter (10) further comprising:
a needle stop (59) mounted on a portion of the injection needle (46) that is positioned within the proximal region (52) of the needle passage (47), the needle stop (59) having a distal end that is sized to prevent passage of the portion of the injection needle on which the needle stop is mounted from passing into the distal region (55) of the needle passage (47);
whereby the needle stop (59) limits the distance that the injection needle (46) can be extended beyond the distal end of the catheter body (12).

11. An injection catheter according to claim 10, wherein the needle stop (59) comprises a collar mounted in surrounding relation to the injection needle.

12. An injection catheter according to claim 11, wherein the collar has a length ranging from about 0.25 mm (0.01 inch) to about 19.1 mm (0.75 inch).

13. An injection catheter according to claim 10, wherein the needle stop (59) comprises an elongated tube (63) mounted in surrounding relation to the injection needle (46).

14. An injection catheter according to claim 13, wherein the elongated tube (63) has a length of at least about 50.8 mm (2 inches).

15. An injection catheter according to claim 10, wherein the needle stop (59) is integral with the injection needle (46).

16. An injection catheter according to claim 10, wherein the injection needle (46) comprises:
a proximal tubing (65) having proximal and distal ends and an outer diameter greater than the distal diameter of the needle passage (47); and
a distal tubing (67) having proximal and distal ends;
wherein the distal end of the proximal tubing (65) is attached to the proximal end of the distal tubing (67).

17. An injection catheter according to claim 10, wherein at least a portion of the needle (46) is surrounded by a protective tubing (47) that forms at least a portion of the needle passage (47).

18. An injection catheter according to claim 10, further comprising a tip electrode (36) mounted on the distal end of the tip section (14), wherein the injection needle (46) extends through a passage (51) in the tip electrode (36).

19. An injection catheter according to claim 1, wherein the tip section (14) has a needle passage (47) extending therethough, the needle passage (47) having a proximal region (52) having a proximal diameter and a distal region (55) having a distal diameter less than the proximal diameter;
wherein the distal end of the injection needle (46) lies within the needle passage (47), the injection needle (46) being longitudinally slidable within the tip section (14) so that its distal end can extend beyond the distal end of the tip section upon suitable manipulation of the needle control handle (17), the injection catheter (10) further comprising:
a needle stop (59) mounted on a portion of the injection needle (46) that is positioned within the proximal region (52) of the needle passage (47), the needle stop (59) having a distal end that is sized to prevent passage of the portion of the injection needle on which the needle stop is mounted from passing into the distal region (55) of the needle passage (47);
whereby the needle stop (59) limits the distance that the injection needle (46) can be extended beyond the distal end of the tip section (14).

20. An injection catheter according to claim 1, wherein the injection needle (46) is longitudinally slidable within the tip section (14) so that its distal end can extend beyond the distal end of the tip section (14) upon suitable manipulation of the needle control handle (17), and further wherein the injection needle (46) comprises one or more pieces of tubing (65, 67), at least one of which comprises plastic.

21. An injection catheter according to claim 20, wherein the injection needle (46) consists essentially of a single piece of plastic tubing.

22. An injection catheter according to claim 20, wherein the injection needle (46) comprises a piece of plastic tubing attached at one of its ends to one end of a piece of metal tubing.

23. An injection catheter according to claim 20, wherein the injection needle (46) has a distal region comprising plastic tubing having straight position memory.

24. An injection catheter according to claim 20, wherein the injection needle (46) has a distal region comprising metal tubing having straight position memory.

25. An injection catheter according to claim 20, wherein the injection needle (46) is provided with a lubricious coating on its outer surface.

26. An injection catheter according to claim 1, wherein the injection needle (46) comprises elongated tubing extending through the tip section (14), catheter body (12), and needle control handle (17) and has a proximal end attached to the needle control handle (17), a distal region within the tip section (14), and an open distal end, wherein the injection needle (46) is longitudinally slidable within the tip section (14) so that its distal region can extend beyond the distal end of the tip section upon suitable manipulation of the needle control handle, and further wherein the distal region of the injection needle (46) has at least one fluid port (45) along its length;
whereby in use, fluid passes out of the needle (46) through the open distal end and the at least one fluid port (45) along the length of the distal region.

27. An injection catheter according to claim 26, wherein the distal region of the injection needle (46) has at least 3 fluid ports (45) along its length.

28. An injection catheter according to claim 26, wherein the distal region of the injection needle (46) has a plurality of fluid ports (45) on only one side.

29. An injection catheter according to claim 26, wherein the distal region of the injection needle (46) has a plurality of fluid ports (45) about its circumference.

## Patentansprüche

1. Injektionskatheter (10) umfassend:
einen Katheterkörper (12) umfassend eine flexible Röhre mit einem proximalen und einem distalen Ende und wenigstens einem Hohlraum (18) dadurch hindurch;
einen Spitzenabschnitt (14) umfassend eine flexible Röhre mit einem proximalen und einem distalen Ende, wobei das proximale Ende des Spitzenabschnittes an dem distalen Ende des Katheterkörpers angebracht ist;
einen Nadelkontrollgriff (17) am proximalen Ende des Katheterkörpers (12), wobei der Nadelkontrollgriff umfasst:
einen Außenkörper (80) mit einer Kolbenkammer (82) darin;
einen Kolben (84) mit zwei Enden, wobei ein Ende des Kolbens innerhalb der Kolbenkammer (82) verschiebbar angebracht ist; und
eine Druckfeder (88) in der Kolbenkammer zwischen dem Außenkörper (80) und dem Kolben (84);
eine Injektionsnadel (46), die sich durch den Spitzenabschnitt (14), den Katheterkörper (12) und den Nadelkontrollgriff (17) erstreckt und ein proximales Ende, das an entweder dem Außenkörper (80) oder dem Kolben (84) angebracht ist, und ein distales Ende innerhalb des distalen Ende des Spitzenabschnittes (14) aufweist;
wobei Kraft in Längsrichtung, die an entweder dem Außenkörper (80) oder dem Kolben (84) angelegt ist, eine Bewegung des jeweils anderen des Außenkörpers (80) und des Kolbens (84) in Längsrichtung verursachen wird, wodurch die Druckfeder (88) zusammengedrückt wird, was zu einer distalen Bewegung der Injektionsnadel (46) relativ zum Katheterkörper (12) führt, so dass sich das distale Ende der Injektionsnadel außerhalb des distalen Endes des Spitzenabschnittes (14) erstreckt.

2. Injektionskatheter nach Anspruch 1, wobei der Kolben (84) weiter umfasst:
einen Schlitz (100), der sich in Längsrichtung entlang eines Teils seiner Länge erstreckt; und
ein Sicherungsmittel (102), das sich durch wenigstens einen Teil des Außenkörpers (80) und in den Schlitz (100) erstreckt;
wobei, während des Betriebs, das Sicherungsmittel (102) die Bewegung des Kolbens (84) relativ zum Außenkörper (80) in Längsrichtung beschränkt.

3. Injektionskatheter nach Anspruch 1, wobei der Nadelkontrollgriff (17) weiter einen axialen Durchgang (83, 85) aufweist, der sich entlang der Länge des Nadelkontrollgriffs erstreckt, durch den sich die Injektionsnadel (46) erstreckt.

4. Injektionskatheter nach Anspruch 3, wobei der Nadelkontrollgriff (17) weiter umfasst:
ein erstes steifes Rohr (40), das fest an dem Kolben (84) angebracht ist und sich durch den axialen Durchgang (83, 85) erstreckt;
ein zweites steifes Rohr (91) mit einem ersten und einem zweiten Ende, das sich durch den axialen Durchgang (83, 85) erstreckt, wobei das erste Ende des zweiten steifen Rohres weiter koaxial in oder um wenigstens einen Teil des ersten steifen Rohres (90) weiter verschiebbar angebracht ist, und das zweite Ende des zweiten Rohres (91) fest an dem Außenkörper (80) angebracht ist; und
wobei sich die Injektionsnadel (46) durch das erste steife Rohr (90) und das zweite steife Rohr (91) erstreckt.

5. Injektionskatheter nach Anspruch 1, wobei wenigstens ein Teil des Kolbens (84) eine mit einem Gewinde versehene äußere Oberfläche (104) aufweist, und wobei der Nadelkontrollgriff (17) weiter eine Daumensteuerung (106) aufweist, die drehbar auf dem Kolben (84) angebracht ist und eine mit einem Gewinde versehene innere Oberfläche (108) aufweist, die mit der mit einem Gewinde versehenen äußeren Oberfläche (104) des Kolbens wechselwirkt;
wobei das Drehen der Daumensteuerung (106) um den Kolben (84) die Position der Daumensteuerung relativ zum Kolben bewegt und wobei weiter die Position der Daumensteuerung (106) die Bewegung des Kolbens (84) relativ zum Außenkörper (80) in Längsrichtung beschränkt.

6. Injektionskatheter nach Anspruch 5, weiter umfassend ein Sicherungsmittel (110), das sich radial durch die Daumensteuerung (106) erstreckt, wobei das Sicherungsmittel (110) gegen die mit einem Gewinde versehene äußere Oberfläche (104) des Kolbens (84) bewegt werden kann, um die Position der Daumensteuerung (106) relativ zu dem Kolben (84) zu sichern.

7. Injektionskatheter nach einem der vorangehenden Ansprüche, wobei die Kolbenkammer (82) ein proximales und ein distales Ende aufweist, das distale Ende des Kolbens (84) verschiebbar innerhalb der Kolbenkammer (82) angebracht ist, wobei das proximale Ende der Injektionsnadel (46) an dem Kolben angebracht ist und wobei weiter die Kraft in Längsrichtung eine distale Kraft umfasst, die an dem Kolben angelegt ist, wobei die distale Kraft eine distale Bewegung des Kolbens (84) relativ zu dem Außenkörper (80) verursacht, was die Druckfeder (88) komprimiert, was zu einer distalen Bewegung der Injektionsnadel (46) relativ zum Außenkörper (80), dem Katheterkörper (12) und dem Spitzenabschnitt (14) führt, so dass sich das distale Ende der Injektionsnadel (46) außerhalb des distalen Endes des Spitzenabschnittes (14) erstreckt und wobei weiter, nach Entfernen der distalen Kraft, sich die Druckfeder (88) expandiert, um eine Kraft auf den Kolben (84) auszuüben, was eine proximale Bewegung des Kolbens (84) relativ zum Außenkörper (80) bedingt, wodurch das distale Ende der Injektionsnadel (46) in das distale Ende des Spitzenabschnittes (14) zurückgezogen wird.

8. Injektionskatheter nach Anspruch 7, wobei die Druckfeder (88) zwei Enden aufweist, wobei ein Ende der Druckfeder in Kontakt mit dem Außenkörper (80) und das andere Ende der Druckfeder in Kontakt mit dem Kolben (84) ist.

9. Injektionskatheter nach Anspruch 7, wobei sich das proximale Ende des Kolbens (84) aus dem proximalen Ende des Außenkörpers (80) erstreckt und eine Daumenrast (101) umfasst, um die manuelle Bewegung des Kolbens (84) relativ zum Außenkörper (80) zu erleichtern.

10. Injektionskatheter nach Anspruch 1, wobei der wenigstens eine Hohlraum (18), der sich durch den Katheterkörper (12) erstreckt, wenigstens einen Nadeldurchgang (47) umfasst, wobei der Nadeldurchgang (47) eine proximale Region (52) mit einem proximalen Durchmesser und eine distale Region (55) mit einem distalen Durchmesser aufweist, der geringer ist als der proximale Durchmesser,
wobei sich die Injektionsnadel (46) durch den Katheterkörper (12) erstreckt und ein distales Ende innerhalb des Nadeldurchgangs (47) aufweist, die Injektionsnadel (46) innerhalb des Katheterkörpers (12) in Längsrichtung verschiebbar ist, so dass sich ihr distales Ende über das distale Ende des Katheterkörpers (12) hinaus erstrecken kann, wobei der Injektionskatheter (10) weiter umfasst:
einen Nadelanschlag (59), der an einem Teil der Injektionsnadel (46) angebracht ist, der innerhalb der proximalen Region (52) des Nadeldurchgangs (47) angeordnet ist, wobei der Nadelanschlag (59) ein distales Ende aufweist, das so dimensioniert ist, dass ein Durchgang des Teils der Injektionsnadel, an dem der Nadelanschlag angebracht ist, am Durchtreten in die distale Region (55) des Nadeldurchgangs (47) gehindert wird;
wobei der Nadelanschlag (59) die Entfernung beschränkt, über die sich die Injektionsnadel (46) über das distale Ende des Katheterkörpers (12) hinaus erstrecken kann.

11. Injektionskatheter nach Anspruch 10, wobei der Nadelanschlag (59) eine Schulter umfasst, die die Injektionsnadel umgebend angebracht ist.

12. Injektionskatheter nach Anspruch 11, wobei die Schulter eine Länge aufweist, die von etwa 0,25 mm (0,01 Zoll) bis etwa 19,1 mm (0,75 Zoll) reicht.

13. Injektionskatheter nach Anspruch 10, wobei der Nadelanschlag (59) ein verlängertes Rohr (63) umfasst, das die Injektionsnadel (46) umgebend angebracht ist.

14. Injektionskatheter nach Anspruch 13, wobei das verlängerte Rohr (63) eine Länge von wenigstens etwa 50,8 mm (2 Zoll) aufweist.

15. Injektionskatheter nach Anspruch 10, wobei der Nadelanschlag (59) einstückig mit der Injektionsnadel (46) ausgebildet ist.

16. Injektionskatheter nach Anspruch 10, wobei die Injektionsnadel (46) umfasst:
eine proximale Röhre (65) mit einem proximalen und einem distalen Ende und einem Außendurchmesser, der größer ist als der distale Durchmesser des Nadeldurchganges (47), und
eine distale Röhre (67) mit einem proximalen und einem distalen Ende;
wobei das distale Ende der proximalen Röhre (65) an das proximale Ende der distalen Röhre (67) angebracht ist.

17. Injektionskatheter nach Anspruch 10, wobei wenigstens ein Teil der Nadel (46) durch eine Schutzröhre (47) umgeben ist, die wenigstens einen Teil des Nadeldurchganges (47) ausbildet.

18. Injektionskatheter nach Anspruch 10, weiter umfassend eine Spitzenelektrode (36), die an dem distalen Ende des Spitzenabschnittes (14) angebracht ist, wobei sich die Injektionsnadel (46) durch einen Durchgang (51) in der Spitzenelektrode (36) erstreckt.

19. Injektionskatheter nach Anspruch 1, wobei der Spitzenabschnitt (14) einen Nadeldurchgang (47) aufweist, der sich dadurch hindurch erstreckt, wobei der Nadeldurchgang (47) eine proximale Region (52) mit einem proximalen Durchmesser und eine distale Region (55) mit einem distalen Durchmesser aufweist, der geringer ist als der proximale Durchmesser;
wobei das distale Ende der Injektionsnadel (46) innerhalb des Nadeldurchganges (47) liegt, die Injektionsnadel (46) in Längsrichtung innerhalb des Spitzenabschnittes (14) verschiebbar ist, so dass sich ihr distales Ende über das distale Ende des Spitzenabschnittes nach geeigneter Handhabung des Nadelkontrollgriffs (17) hinaus erstrecken kann, wobei der Injektionskatheter weiter umfasst:
einen Nadelanschlag (59), der an einem Teil der Injektionsnadel (46) angebracht ist, der innerhalb der proximalen Region (52) des Nadeldurchganges (47) angeordnet ist, wobei der Nadelanschlag (59) ein distales Ende aufweist, das so dimensioniert ist, dass ein Durchgang des Teils der Injektionsnadel, an dem der Nadelanschlag angebracht ist, am Durchtreten in die distale Region (55) des Nadeldurchganges (47) gehindert wird;
wobei der Nadelanschlag (59) die Entfernung begrenzt, über die sich die Injektionsnadel (46) über das distale Ende des Spitzenabschnittes (14) hinaus erstrecken kann.

20. Injektionskatheter nach Anspruch 1, wobei die Injektionsnadel (46) in Längsrichtung innerhalb des Spitzenabschnittes (14) verschiebbar ist, so dass sich ihr distales Ende über das distale Ende des Spitzenabschnittes (14) nach geeigneter Handhabung des Nadelkontrollgriffs (17) hinaus erstrecken kann, und wobei weiter die Injektionsnadel (46) ein oder mehrere Röhrenstücke (65, 67) aufweist, von denen wenigstens eines Kunststoff umfasst.

21. Injektionskatheter nach Anspruch 20, wobei die Injektionsnadel (46) im Wesentlichen aus einer einzelstückigen Kunststoffröhre besteht.

22. Injektionskatheter nach Anspruch 20, wobei die Injektionsnadel (46) ein Stück Kunststoffröhre umfasst, das an einem von ihren Enden an einem Ende eines Metallröhrenstückes angebracht ist.

23. Injektionskatheter nach Anspruch 20, wobei die Injektionsnadel (46) eine distale Region aufweist, die eine Kunststoffröhre mit einem Gedächtnis für eine gerade Position umfasst.

24. Injektionskatheter nach Anspruch 20, wobei die Injektionsnadel (46) eine distale Region aufweist, die eine Metallröhre mit einem Gedächtnis für eine gerade Position umfasst.

25. Injektionskatheter nach Anspruch 20, wobei die Injektionsnadel (46) mit einer schmierfähigen Beschichtung auf ihrer äußeren Oberfläche versehen ist.

26. Injektionskatheter nach Anspruch 1, wobei die Injektionsnadel (46) eine verlängerte Röhre, die sich durch den Spitzenabschnitt (14), den Katheterkörper (12) und den Nadelkontrollgriff (17) erstreckt und ein proximales Ende aufweist, das an dem Nadelkontrollgriff (17) angebracht ist, eine distale Region innerhalb des Spitzenabschnittes (14) und ein offenes distales Ende umfasst, wobei die Injektionsnadel (46) in Längsrichtung innerhalb des Spitzenabschnittes (14) verschiebbar ist, so dass sich ihre distale Region über das distale Ende des Spitzenabschnittes nach geeigneter Handhabung des Nadelkontrollgriffs (17) hinaus erstrecken kann und wobei weiter die distale Region der Injektionsnadel (46) wenigstens eine Flüssigkeitsöffnung (45) entlang ihrer Länge aufweist;
wobei, bei der Anwendung, Flüssigkeit aus der Nadel (46) durch das offene distale Ende und die wenigstens eine Flüssigkeitsöffnung (45) entlang der Länge der distalen Region austritt.

27. Injektionskatheter nach Anspruch 26, wobei die distale Region der Injektionsnadel (46) wenigstens drei Flüssigkeitsöffnungen (45) entlang ihrer Länge aufweist.

28. Injektionskatheter nach Anspruch 26, wobei die distale Region der Injektionsnadel (46) eine Vielzahl von Flüssigkeitsöffnungen (45) auf nur einer Seite aufweist.

29. Injektionskatheter nach Anspruch 26, wobei die distale Region der Injektionsnadel (46) eine Vielzahl von Flüssigkeitsöffnungen (45) um ihren Umfang aufweist.

## Revendications

1. Cathéter à injection (10) comprenant :
un corps de cathéter (12) comprenant une tubulure flexible ayant des extrémités proximale et distale et au moins une lumière (18) à travers celle-ci ;
une section d'embout (14) comprenant une tubulure flexible ayant des extrémités proximale et distale, dans lequel l'extrémité proximale de la section d'embout est montée sur l'extrémité distale du corps de cathéter ;
une poignée de commande de l'aiguille (17) au niveau de l'extrémité proximale du corps de cathéter (12), la poignée de commande de l'aiguille comprenant :
un corps extérieur (80) ayant une chambre de piston (82) dans celui-ci ;
un piston (84) ayant deux extrémités, dans lequel une extrémité du piston est montée de manière coulissante à l'intérieur de la chambre de piston (82) ; et
un ressort de compression (88) dans la chambre de piston entre le corps extérieur (80) et le piston (84) ;
une aiguille à injection (46) s'étendant à travers la section d'embout (14), le corps de cathéter (12) et la poignée de commande de l'aiguille (17) et ayant une extrémité proximale attachée soit au corps extérieur (80) soit au piston (84) et une extrémité distale à l'intérieur de l'extrémité distale de la section d'embout (14) ;
moyennant quoi une force longitudinale appliquée soit au corps extérieur (80) soit au piston (84) entraîne un mouvement longitudinal de l'autre de soit le corps extérieur soit le piston , compressant de cette manière le ressort de compression (88), entraînant un mouvement distal de l'aiguille à injection (46) par rapport au corps de cathéter (12), de sorte que l'extrémité distale de l'aiguille à injection s'étende à l'extérieur de l'extrémité distale de la section d'embout (14).

2. Cathéter à injection selon la revendication 1, dans lequel le piston (84) comprend en outre :
une fente (100) s'étendant longitudinalement le long d'une portion de sa longueur ; et
des moyens de fixation (102) s'étendant à travers au moins une portion du corps extérieur (80) et dans la fente (100) ;
moyennant quoi, pendant le fonctionnement, les moyens de fixation (102) limitent le mouvement longitudinal du piston (84) par rapport au corps extérieur (80).

3. Cathéter à injection selon la revendication 1, dans lequel la poignée de commande de l'aiguille (17) comprend en outre un passage axial (83, 85) s'étendant le long de la longueur de la poignée de commande de l'aiguille à travers laquelle s'étend l'aiguille à injection (46).

4. Cathéter à injection selon la revendication 3, dans lequel la poignée de commande de l'aiguille (17) comprend en outre :
un premier tube rigide (90) fixé à demeure au piston (84) et s'étendant à travers le passage axial (83, 85) ;
un second tube rigide (91) ayant des première et seconde extrémités s'étendant à travers le passage axial (83, 85), dans lequel la première extrémité du second tube rigide est en outre montée de manière coulissante coaxialement dans ou autour au moins d'une partie du premier tube rigide (90), et la seconde extrémité du second tube rigide (91) est montée à demeure sur le corps extérieur (80) ; et
dans lequel l'aiguille à injection (46) s'étend à travers le premier tube rigide (90) et le second tube rigide (91).

5. Cathéter à injection selon la revendication 1, dans lequel au moins une portion du piston (84) a une surface extérieure filetée (104), et dans lequel la poignée de commande de l'aiguille (17) comprend en outre une commande de pouce (106) montée de manière rotative sur le piston (84) et ayant une surface intérieure filetée (108) qui interagit avec la surface extérieure filetée (104) du piston ;
moyennant quoi la rotation de la commande de pouce (106) autour du piston (84) déplace la position de la commande de pouce par rapport au piston , et moyennant quoi en outre la position de la commande de pouce (106) limite le mouvement longitudinal du piston (84) par rapport au corps extérieur (86).

6. Cathéter à injection selon la revendication 5, comprenant en outre des moyens de fixation (110) s'étendant radialement à travers la commande de pouce (106), moyennant quoi les moyens de fixation (110) peuvent être déplacés contre la surface extérieure filetée (104) du piston (84) pour fixer la position de la commande de pouce (106) par rapport au piston (84).

7. Cathéter à injection selon l'une quelconque des revendications précédentes, dans lequel la chambre de piston (82) a des extrémités proximale et distale, l'extrémité distale du piston (84) étant montée de manière coulissante à l'intérieur de la chambre de piston (82) dans lequel l'extrémité proximale de l'aiguille à injection (46) est fixée au piston et dans lequel en outre la force longitudinale comprend une force distale appliquée au piston , la force distale entraînant un mouvement distal du piston (84) par rapport au corps extérieur (80), comprimant le ressort de compression (88), entraînant un mouvement distal de l'aiguille à injection (46) par rapport au corps extérieur (80), au corps de cathéter (12) et à la section d'embout (14) de sorte que l'extrémité distale de l'aiguille à injection (46) s'étende à l'extérieur de l'extrémité distale de la section d'embout (14) et moyennant quoi en outre, par un retrait de la force distale, le ressort de compression (88) se dilate pour exercer une force sur le piston (84), entraînant un mouvement proximal du piston (84) par rapport au corps extérieur (80), rétractant de cette manière l'extrémité distale de l'aiguille à injection (46) dans l'extrémité distale de la section d'embout (14).

8. Cathéter à injection selon la revendication 7, dans lequel le ressort de compression (88) a deux extrémités, dans lequel une extrémité du ressort de compression est en contact avec le corps extérieur (80) et l'autre extrémité du ressort de compression est en contact avec le piston (84).

9. Cathéter à injection selon la revendication 7, dans lequel l'extrémité proximale du piston (84) s'étend à l'extérieur de l'extrémité proximale du corps extérieur (80) et comprend un appui-pouce (106) pour faciliter le mouvement manuel du piston (84) par rapport au corps extérieur (80).

10. Cathéter à injection selon la revendication 1, dans lequel la au moins une lumière (18) s'étendant à travers le corps de cathéter (12) comprend au moins un passage d'aiguille (47), le passage d'aiguille (47) ayant une région proximale (52) ayant un diamètre proximal et une région distale (55) ayant un diamètre distal inférieur au diamètre proximal, dans lequel l'aiguille à injection (46) s'étend à travers le corps de cathéter (12) et a une extrémité distale à l'intérieur du passage d'aiguille (47), l'aiguille à injection (46) pouvant coulisser longitudinalement à l'intérieur du corps de cathéter (12) de sorte que son extrémité distale s'étende au-delà de l'extrémité distale du corps de cathéter (12), le cathéter à injection (10) comprenant en outre :
une arrêt de l'aiguille (59) monté sur une portion de l'aiguille à injection (46) qui est positionnée à l'intérieur de la région proximale (52) du passage d'aiguille (47), l'arrêt de l'aiguille (57) ayant une extrémité distale dont la taille est adaptée pour empêcher le passage de la portion de l'aiguille à injection sur laquelle l'arrêt de l'aiguille est monté de passer dans la région distale (55) du passage d'aiguille (47),
moyennant quoi l'arrêt de l'aiguille (57) limite la distance sur laquelle l'aiguille à injection (46) peut être étendue au-delà de l'extrémité distale du corps de cathéter (12).

11. Cathéter à injection selon la revendication 10, dans lequel l'arrêt de l'aiguille (59) comprend un collier monté autour de l'aiguille à injection.

12. Cathéter à injection selon la revendication 11, dans lequel le collier a une longueur comprise entre environ 0,25 mm (0,01 pouce) et environ 19,1 mm (0,75 pouce).

13. Cathéter à injection selon la revendication 10, dans lequel l'arrêt de l'aiguille (59) comprend un tube allongé (63) monté autour de l'aiguille à injection (46).

14. Cathéter à injection selon la revendication 13, dans lequel le tube allongé (63) a une longueur d'au moins environ 50,8 mm (2 pouces).

15. Cathéter à injection selon la revendication 10, dans lequel l'arrêt de l'aiguille (59) fait partie intégrante de l'aiguille à injection (46).

16. Cathéter à injection selon la revendication 10, dans lequel l'aiguille à injection comprend :
une tubulure proximale (65) ayant des extrémités proximale et distale et un diamètre extérieur supérieur au diamètre distal du passage d'aiguille (47), et
une tubulure distale (67) ayant des extrémités proximale et distale ;
dans lequel l'extrémité distale de la tubulure proximale (65) est fixée à l'extrémité proximale de la tubulure distale (61).

17. Cathéter à injection selon la revendication 10, dans lequel au moins une portion de l'aiguille (46) est entourée par une tubulure de protection (47) qui forme au moins une portion du passage d'aiguille (47).

18. Cathéter à injection selon la revendication 10, comprenant en outre une électrode d'embout (36) montée sur l'extrémité distale de la section d'embout (14), dans lequel l'aiguille à injection (46) s'étend à travers un passage (51) dans l'électrode d'embout (36).

19. Cathéter à injection selon la revendication 1, dans lequel la section d'embout (14) a un passage d'aiguille (47) s'étendant à travers celle-ci, le passage d'aiguille (47) ayant une région proximale (52) ayant un diamètre proximal et une région distale (55) ayant un diamètre distal inférieur au diamètre proximal ;
dans lequel l'extrémité distale de l'aiguille à injection (46) se situe à l'intérieur du passage d'aiguille (47), l'aiguille à injection (46) pouvant coulisser longitudinalement à l'intérieur de la section d'embout (14) de sorte que l'extrémité distale puisse s'étendre au-delà de l'extrémité distale de la section d'embout par une manipulation convenable de la poignée de commande de l'aiguille (117), le cathéter à injection (10) comprenant en outre :
une arrêt de l'aiguille (59) monté sur une portion de l'aiguille à injection (46) qui est positionnée à l'intérieur de la région proximale (52) du passage d'aiguille (47), l'arrêt de l'aiguille (59) ayant une extrémité distale dont la taille est adaptée pour empêcher le passage de la portion de l'aiguille à injection sur laquelle l'arrêt de l'aiguille est monté de passer dans la région distale (55) du passage d'aiguille (47),
moyennant quoi l'arrêt de l'aiguille (59) limite la distance sur laquelle l'aiguille à injection (46) peut être étendue au-delà de l'extrémité distale de la section d'embout (14).

20. Cathéter à injection selon la revendication 1, dans lequel l'aiguille à injection (46) peut coulisser longitudinalement à l'intérieur de la section d'embout (14) de sorte que son extrémité distale puisse s'étendre au-delà de l'extrémité distale de la section d'embout (14) par une manipulation convenable de la poignée de commande de l'aiguille (17) et dans lequel en outre l'aiguille à injection (46) comprend une pièce ou plus de tubulure (65, 67), au moins de ceux-ci comprenant du plastique.

21. Cathéter à injection selon la revendication 20, dans lequel l'aiguille à injection (46) se compose essentiellement d'une pièce unique de tubulure de plastique.

22. Cathéter à injection selon la revendication 20, dans lequel l'aiguille à injection comprend une pièce de tubulure de plastique fixée au niveau d'une de ses extrémités à une extrémité d'une pièce de tubulure métallique.

23. Cathéter à injection selon la revendication 20, dans lequel l'aiguille à injection (46) a une région distale comprenant une tubulure de plastique ayant une mémoire de position droite.

24. Cathéter à injection selon la revendication 20, dans lequel l'aiguille à injection (46) a une région distale comprenant une tubulure métallique ayant une mémoire de position droite.

25. Cathéter à injection selon la revendication 20, dans lequel l'aiguille à injection (46) est fournie avec un revêtement lubrifiant sur sa surface extérieure.

26. Cathéter à injection selon la revendication 1, dans lequel l'aiguille à injection (46) comprend une tubulure allongée s'étendant à travers la section d'embout (14), le corps de cathéter (12) et la poignée de commande de l'aiguille (17), et a une extrémité proximale fixée à la poignée de commande de l'aiguille (17), une région distale à l'intérieur de la section d'embout (14) et une extrémité distale ouverte, dans lequel l'aiguille à injection (46) peut coulisser longitudinalement à l'intérieur de la section d'embout (14) de sorte que sa région distale puisse s'étendre au-delà de l'extrémité distale de la section d'embout par une manipulation convenable de la poignée de commande de l'aiguille (17), et dans lequel en outre la région distale de l'aiguille à injection (46) a au moins un orifice de fluide (45) le long de sa longueur ;
moyennant quoi, pendant l'utilisation, le fluide sort de l'aiguille (46) à travers l'extrémité distale ouverte et le au moins un orifice de fluide (45) le long de la longueur de la région distale.

27. Cathéter à injection selon la revendication 26, dans lequel la région distale de l'aiguille à injection (46) a au moins 3 orifices de fluide (45) le long de sa longueur.

28. Cathéter à injection selon la revendication 26, dans lequel la région distale de l'aiguille à injection (46) a une pluralité d'orifices de fluide (45) sur seulement un côté.

29. Cathéter à injection selon la revendication 26, dans lequel la région distale de l'aiguille à injection (41) a une pluralité d'orifices de fluide (45) autour de sa circonférence.
